# EUROPEAN PATENT SPECIFICATION

(11) **EP 1 711 620 B1**
(45) Date of publication and mention of the grant of the patent: **13.05.2009**
(21) Application number: 04806774.8
(22) Date of filing: 22.12.2004
(51) Int. Cl.: C12Q 1/68

(54) **METHODS FOR DETECTION OF MYCOBACTERIUM TUBERCULOSIS**
VERFAHREN ZUM NACHWEIS VON MYCOBACTERIUM TUBERCULOSIS
PROCEDES DE DETECTION DU BACILLE DE KOCH

(30) Priority: 23.12.2003 IN DE15992003
(43) Date of publication of application: 18.10.2006
(73) Proprietor: All India Institute of Medical Sciences, Ansari Nagar New Delhi 110029 (IN); Department of Biotechnology (a Department of the Government of India), New Delhi 110 003 (IN)
(72) Inventor: SINGH, Sarman, Dept. of Laboratory Medicine, Ansari Nagar, NewDelhi 110029 (IN); SHARMA, Pawan, Int. Centre of Genetic Eng and Bio, New Delhi 110 029 (IN)
(74) Representative: Winter, Brandl, Fürniss, Hübner Röss, Kaiser, Polte Partnerschaft Patent- und Rechtsanwaltskanzlei
(86) International application number: PCT/IN2004/000396
(87) International publication number: WO 2005/061730

(56) References cited:
- RU-C1- 2 163 638
- DATABASE GENBANK [Online] 23 October 2001 SINGH G. ET AL., XP002999476 Retrieved from stn Database accession no. AF420491

## Description

### TECHNICAL FIELD

The invention provides novel oligonucleotide primers for the amplification of Early Secretory Antigenic Target (ESAT)-6 regions for detection of *Mycobacterium* species. The primers are used for differentiating the *Mycobacterium tuberculosis* from other species of *Mycobacterium.* Further, the invention provides a method for detection of *Mycobacterium tuberculosis* based on the DNA amplification of the ESAT-6 region.

### BACKGROUND AND PRIOR ART

According to the WHO, tuberculosis still kills 3 million individuals per year, making it the leading infectious cause of death. It is believed that one in every three individuals on the planet harbor the causative microorganism belonging to the genus *Mycobacterium* (13, 24). This genus represents a complex phenotypic and genotypic diversity amongst its more than 100 odd species (4,9,10,14,15,17,27,28,30,34). Though, the most important human pathogenic species is *Mycobacterium tuberculosis* (M.tb), other species commonly known as non- tubercular Mycobacteria (NTM) or Mycobacteria other than tuberculosis (MOTT) also cause human infections in various clinical forms, particularly in immunosuppressed patients, adding to the human suffering in terms of morbidity and mortality. The cases of HIV-TB co-infections are rapidly increasing, after the AIDS epidemic, from both the developed and developing world (2, 10, 13, 18-21, 24). Most of these non-Tubercular Mycobacteria are not susceptible to the conventional anti-tubercular treatment and are wrongly considered due to infection with multi-drug resistant (MDR) strains of *M*. *tuberculosis* (5-7, 10, 21, 26, 31, 32). Many of these MDR labeled infections are actually not caused by the drug resistant strains of *M.tuberculsosis* but by the non- tubercular Mycobacteria. HIV-Mycobacteria co-infection is the most common killer opportunistic infection in Indian AIDS patients (31), but the outbreaks of infections due to the NTM in these severely immunocompromised population, can not be ruled out (19). Hence it is very important to identify the causative agent to the species level for appropriate treatment.

However, in India the diagnosis of Mycobacterial infections is established empirically on clinico-radiological basis or only by sputum smear examination, and the infections caused by non-tubercular Mycobacteria are under-diagnosed due to lack of diagnostic facilities. The speciation of *Mycobacteria* using conventional methods is very slow, labor intensive, hazardous and not always reproducible (33) and hence left unattempted by most of the Indian Laboratories.

To overcome the shortcomings of conventional methods of species specific identification of *Mycobacteria,* the molecular techniques are being more commonly used, in the recent years. Molecular identification methods are rapid, highly sensitive and specific and can be used on a large number of samples (1, 6, 9, 16, 33, US Patent No. 5652106, US Patent No.5731150). One molecular target which has proved to be the most promising and commonly used is the 16S rRNA gene analysis. This gene is conserved in all the species of Mycobacterial genus (1, 15, 16, US Patent No. 5811269). Once the genus mycobacterium is identified, the species can be differentiated by southern hybridization using the species specific probes, or gene sequencing (4, 11, 22, 28). However, identification of all the non-tubercular species of *Mycobacteria* may require several specific primers and repeated experimentation and most often it may not be necessary in a resource poor setting, particularly from the fresh cases, when several species can easily be identified on the basis of phenotypic characters. Therefore, the present invention intends to differentiate the *Mycobacterium tuberculosis* from non-tubercular Mycobacterium species, using a rapid PCR amplification method. For this, a novel, simple and rapid method for differentiating MTB and NTM has been developed, that uses a pair of oligonucleotide primers targeting the *esat-6* and 16S rRNA genes.

The 16S rRNA gene amplification method is a good tool to identify all the *Mycobacteria.* However, its further utility is restricted only for taxonomic purposes after sequencing the amplified gene product. Therefore, the single PCR method can not answer the most important clinical question, whether the Mycobacterium is *M. tuberculosis* or other than *M. tuberculosis.* Therefore, with clinical point of view, it is extremely important that the Clinical Microbiologist not only diagnoses a Mycobacterial infection in his patient but also provides the identification of the etiological agent, whether it is *M tuberculosis* or other species of *Mycobacterium* for which the treatment protocol is totally different from the first. On the other hand, the novel oligonucleotide primer pair designed in the present invention target the *esat-6* gene which is *Mycobacterium tuberculosis* specific gene coding for the early antigen of 6 kDa mass (3,8, 23,25). Though this antigen has been used in the humoral immunodiagnosis as well as for evaluating the cell mediated immune response against *M. tuberculosis,* replacing PPD with this antigen in skin testing (3, 8). More recently, it has also been used for vaccination against tuberculosis (23, 25, US Patent No.6649170) but its gene has never been used as a target for molecular diagnosis of tuberculosis. This gene is deleted in species of Mycobacteria other than tuberculosis complex (4).

The antigen- antibody based test methods have been a research topic but no antigen has been found to be satisfactory. Most of the genus-specific antigens failed because they cross reacted with the environmental *Mycobacteria* and with BCG which is given as a vaccine. Even the *M. tuberculosis* specific antigen (ESAT-6 antigen in this case) has a fundamental drawback of poor predictive value to make an organ specific disease diagnosis. The detection of antibodies against ESAT-6 protein has been found to be somewhat useful in tuberculosis non-endemic countries, its utility in TB endemic countries such as whole of Asia, Africa, Russia and other South American countries is very limited due to sub-clinical exposures of the population. Obviously all the exposed persons will have circulating antibodies in their blood against this antigen. Therefore, if a pre-exposed asymptomatic person (antibodies already positive) gets fresh TB brain infection (TB meningitis) and in another pre-exposed person there is no such fresh infection, the antibody detection assays will not be useful for the specific diagnostic use in such cases, as both these patients will be positive for these antibodies. To explain it in other words, antibody detection assays, for diseases of high endemicity (e.g. Tuberculosis which is airborne) have very poor specificity and organ specific diagnosis can not be made at all, as the antibody detection methods are indirect evidences of infection.

On the other hand the molecular methods such as PCR are highly specific because in these methods the genome of the living organism from the specific diseased site is detected. In other words, using PCR amplification, the specific diagnosis of tubercular meningitis, abdominal tuberculosis, gastrointestinal tuberculosis, genitourinary tuberculosis besides the pulmonary tuberculosis can be made. Also the PCR amplification will detect only active diseases and not the old exposures. Moreover the biggest advantage of molecular methods is that the DNA of causative agent (Mycobacterium) can be detected from old samples such as mummies, fossils etc. while the antibodies can not.

Gey van Pittius, N.C. et al. [Genome Biol. 2001,2(10)] analyzes finished and unfinished genome sequencing data of 98 publicly available microbial genomes for the presence of orthologs of the ESAT-6 loci. It is further disclosed that the multiple duplicates of the ESAT-6 gene cluster found in the genome of *M tuberculosis* H37Rv are also conserved in the genomes of other mycobacteria, for example *M tuberculosis* CDC1551, *M tuberculosis* 210, *M bovis, M leprae, M. avium,* and the avirulent strain *M smegmatis.* Phylogenetic analyses of the resulting sequences have established the duplication order of the gene clusters and demonstrated that the gene cluster known as region 4 (Rv3444c-3450c) is ancestral. Region 4 is also the only region for which an ortholog could be found in the genomes of *Corynebacterium diphtheriae* and *Streptomyces coelicolor.* Comparative genomic analysis reveals that the presence of the ESAT-6 gene cluster is a feature of some high-G+C Gram-positive bacteria. Multiple duplications of this cluster have occurred and are maintained only within the genomes of members of the genus *Mycobacterium.*

Berthet, F.-X. et al. (Microbiol. 1998, 144:3195-3203) discloses mapping and characterization of the promoter region controlling the synthesis of the *M. tuberculosis* ESAT-6 antigen. Evidence is provided that *esat-6* is co-transcribed with a novel gene designated *lhp.* The authors identify 13 genes related to the Ihp/esat-6 operon, defining a novel gene family in the M. tuberculosis genome. The M. tuberculosis *lhp* gene product is identified as CFP-10, a low molecular-mass protein found in the short-term culture filtrate. The results disclosed suggest that the genes encoding CFP-10 and ESAT-6 are transcribed together in M. tuberculosis and that both code for small exported proteins.

Hernandez Abanto, S.M. et al. (2000, 14(5): 238-245) discloses evaluation of Henes-PCR (heminested-PCR) assay for *Mycobacterium* detection in different clinical specimens from patients with or without tuberculosis-associated HIV infection. The procedure disclosed is designed to identify *Mycobacterium* spp., *M. tuberculosis* complex (MTC), and M. avium complex (MAC), although it has the potential to include more primers for the identification of other species. Sensitivity and negative predictive value of Henes-PCR are significantly higher than culture procedure for microscopy-negative specimens. These findings suggest that Henes-PCR is a useful test for rapid detection of *Mycobacterium* in clinically suspected cases of tuberculosis with smear-negative results.

The present invention shows its utility in species-specific and rapid molecular diagnosis of tuberculosis using *esat*-6 gene amplification, for the first time.

### Definitions of certain terms used in the specification

- AIDS -: Acquired Immunodeficiency Syndrome;
- M.tb-: *Mycobacterium tuberculosis;*
- NTM-: Non-tubercular Mycobacteria;
- MDR-TB-: Multi drug resistant tuberculosis

### BRIEF DESCRIPTION OF ACCOMPANYING DRAWING

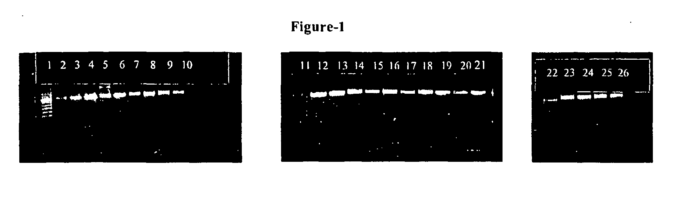

- **Figure 1**: The PCR method for genus-specific identification of the *Mycobacterium* species based on the 16s rRNA gene. Lanes 1, 11, & 22 are 100 bp molecular weight markers. Lanes 2, 12, & 23 are H37rv standard strain of *Mycobacterium tuberculosis.* Lanes 3-10 are standard non-tubercular *Mycobacteria* (as shown in table 1), 13-21 and 24-26 are clinical isolates.

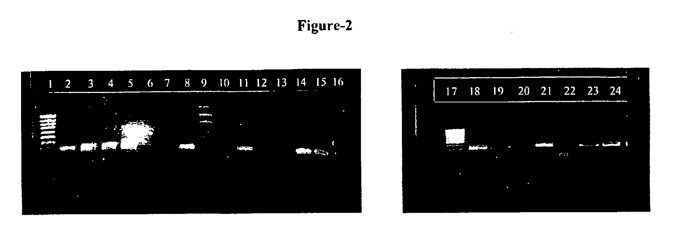

- **Figure 2:**: The isolates which were identified as *Mycobacteria* on the basis of genus-specific 16s rRNA PCR were subjected to amplification based on the ESAT-6 region. Lane I & 17 is 100 bp molecular weight markers. Lane 2 & 18 is H37rv standard strain of *Mycobacterium tuherculosis.* Lane 3-15 & 19-24 is the same clinical isolates as shown in panel A. The lane 16 is *M. bovis* showing no amplification.

### OBJET OF THE INVENTION

The main object of the present invention is to develop an oligonucleotide primer pair for specific amplification of the Early Secretory Antigen of Target *(esat)-6 gene* of *Mycobacterium* species

Another object of the present invention is to develop a method for detecting M. tuberculosis in a sample based on the amplification of *esat-*6 gene using the primer pair.

Still another object of the present invention is to develop a method for detecting M. tuberculosis wherein the amplification is done by polymerase chain reaction.

Further object of the present invention is to develop a diagnostic kit for detecting *M. tuberculocis,* based on the amplification of *(esat)-6* gene.

### SUMMARY OF THE INVENTION

The present invention provides novel oligonucleotide primer pair consisting of SEQ ID NO: 3 and SEQ ID NO: 4 for amplification of Early Secretory Antigenic Target (*esat*)-6-gene of *Mycobacterium* species. The invention also provides a method for detecting *M. tuberculosis* in a sample based on the amplification of *esat-6* gene, comprising isolating DNA template from the sample, amplifying with the above oligonucleotide primer pair and subjecting the amplified DNA product to separation and staining to detect the presence of amplified DNA product for identifying *Mycobacterium tuberculosis* in the sample. The invention further provides a diagnostic kit for detection *of Mycobacterium tuberculosis.* The invention also provides a method of detecting *Mycobacterium tuberculosis* from a sample by amplifying the 16s rRNA region from the isolated DNA template by conventional methods to detect *Mycobacterium* species and further amplifying the positive sample containing *Mycobacterium* species using novel oligonucleotide primer pair for amplification of *ESAT-*6 region of a band of 320 bp which is indicative of the presence of *Mycobacterium tuberculosis.*

### DETAILED DESCRIPTION OF THE INVENTION

In accordance, the present invention provides for an oligonucleotide primer pair for amplification of the Early Secretory Antigen of Target (*esat*)-6 gene consisting of SEQ ID NO: 3 and SEQ ID NO: 4.

In an embodiment, the present invention provides a method for detecting *M. tubercerlosis* using the oligonucleotide primer pair, the said method comprising the steps of:
a) isolating DNA template from the sample,
b) amplifying the DNA template by adding a reaction buffer, oligonucleotide primer pair consisting of SEQ ID NO: 3 and SEQ ID NO: 4, and heat stable DNA polymerase to obtain an amplified DNA product, and
c) subjecting the amplified DNA product of step (b) to separation, and staining to detect the presence of amplified DNA product wherein the presence of amplified DNA product is indicative of *Mycobacterium tuberculosis* in the sample.

Further embodiment of the present invention is for a method for detecting *M. tuberculosis,* based on the amplification of the reaction mixture containing DNA from sample, oligonucleotide primer pair consisting of SEQ ID NO: 3 and SEQ ID NO: 4, reaction buffer, dNTPs and DNA polymerase preferably *Taq* polymerase.

Another embodiment of the present invention is for a method for the detection of *M. tuberculosis* from either a clinical or a culture sample, wherein the clinical samples are selected from sputum, bronchoalveolar lavage fluid, pleural fluid, ascetic/peritoneal fluid, cerebrospinal fluid (CSF), pus, faecal matter, urine, amniotic fluid, menstrual blood, peripheral blood or other body fluids, lymphnode, pus or other aspirate and tissue biopsies.

Further embodiment of the present invention is for a method for detecting *M. tuberculosis* using the oligonucleotide primers consisting of SEQ ID NO: 3, and SEQ ID NO: 4, the DNA sample obtained from Mycobacterial cultures.

Yet another embodiment of the present invention is for a method for detecting *M. tuberculosis* wherein the amplification is done by polymerase chain reaction.

Still another embodiment of the present invention is the presence of the amplified DNA product is of 320 bp in size which is indicative of the *M. tuberculosis.*

In yet another embodiment of the present invention is for a diagnostic kit for detection of *M. tuberculosis,* based on the amplification of *(esat)-6* gene. The kit further comprises of oligonucleotides primer pair consisting of SEQ ID NO: 3 and SEQ ID NO: 4, reaction buffer, DNA polymerase preferably *Taq* polymerase, negative and positive control, DNA marker, deoxyribonucleictriphosphates (dNTPs) and an instruction manual.
A further embodiment of the present invention is for a method for detecting *M.tuberculosis* based on amplification wherein, the said method comprising the steps of:
i. amplifying the 16s rRNA region from the isolated DNA template using the primer pair consisting of SEQ ID NO: 1 and SEQ ID NO: 2 to obtain first amplified product using conventional method,
ii. detecting the amplified product of step (a) wherein the presence of 1030 base pair amplified DNA product is indicative of positive sample for the presence of *Mycobacterium* species,
iii. employing the DNA from the positive samples identified from step (b) for further detection of *M. tuberculosis* based on the amplification of *esat-6* gene,
iv. amplifying the *esat-6* gene using the primer pair consisting of SEQ ID NO: 3 and SEQ ID NO: 4 to obtain second amplified product using method as claimed in claim 2,
v. detecting the amplified product of step (d) wherein the presence of 320 base pair is indicative of *Mycobacterium tuberculosis* in the sample and absence is indicative of *Mycobacterium* species other than *M.tuberculusis.*

The present invention teaches a method for detection of *M.tuberculosis* based on amplification of *esat*-6 gene. This invention provide the novel oligonucleotide primer pair & the method of detection which is given below: Two hundred and sixty five patients were taken up for this study for detection of Mycobacterial infection. Their clinical and laboratory data were analyzed to ascertain the sensitivity of various diagnostic methods (Table 1). Only 85 patients (32.0%) showed the presence of *Mycobacteria* in their L-J culture (See Example 1 for culturing of *Mycobacterium).* Other patients whose cultures remained negative after 10 weeks of incubation were excluded from this study. These 85 patients could be divided into four groups on the basis of clinical disease manifestations and their HIV status. 70 patients were HIV negative and 15 were HIV-1 positive. Out of the 70 HIV negative patients, 24 patients were present with lymphadenitis, and the remaining 46 had pulmonary manifestation. Of these only 22 were new cases who had not received any anti-tubercular treatment while the other 24 were bacteriologically confirmed cases of pulmonary tuberculosis and were on anti-tubercular treatment but without response. Because, these patients continued to excrete the acid fast bacilli in their sputum even after 6 months of treatment with Isoniazide, Rifampicin, Ethambutol and Pyrazinamide, these cases were labeled as (Multi Drug Resistance) MDR patients. The mean month time for anti-tubercular treatment in these patients was 26.7 ± 17, the maximum being 6 years. All HIV infected patients included in this study were present with pulmonary manifestations.

Overall the detection rate of L-J culture in the applicant's laboratory was 32% (85/265). Similarly, the detection rate of Ziehl-Neelson (Z-N) staining was only 19.6%, and that of Auramine-O (AO) staining 22.3%.(As given in Example 2) However, the sensitivity of AO staining and ZN stained smears on culture proven cases was 75% and 66.6% for MDR cases, 72.7% and 68.2% for new pulmonary tuberculosis cases, 66.6% and 58.3% for tubercular lymphadenitis and 60% and 46.6% in HIV infected pulmonary tuberculosis cases, respectively. Hence the AO stain had a clear edge over Z-N stain and the culture had the highest detection rate.

On culture examination, 86 strains could be isolated from the 85 samples. (For details see example 1& 2) One sample grew two types of colonies one being a rapid grower. All the 86 cultures which were identified as Mycobacterial in nature using standard protocol such as culture examination as exemplified in Example 2.

### Genus-specific PCR assay

These cultures were further tested for the presence of Mycobacterium species using molecular tools. The DNA was isolated directly from the above 86 cultures. The DNA isolation was carried out as given in Example 3. All the 86 DNA samples were subjected to genus-specific amplification as given in Example 4. Briefly, DNA from the samples was amplified using 16S rRNA gene primers (genus-specific primers) using suitable buffers. These primers (genus-specific primers) are given below.
Primer 16S rRNA 285: 5' gag agt ttg atc ctg get cag 3' (SEQ ID NO: 1) and
Primer 16S rRNA 264: 5' tgc aca aca ggc cac aag gga 3. (SEQ ID NO: 2)

After amplification using the above primers the amplified products were separated on 0.9% agarose gel and stained using ethidium bromide. All the samples showed a product of size 1030 bp .Figure 1 shows a representative profile of the PCR assay of some of the samples analaysed All the lanes showed an amplified product of size 1030 bp indicating the presence of *Mycobacterium* species.. This indicated a positive result clearly showing the presence of *mycobacterium* strains as seen in Figure 1. Some of the amplified products were sequenced (29) to confirm whether these sequences are of *Mycobacterial* origin. From the sequence analysis it is clear that all the strains were *Mycobacterium* strains and the details are given in Example 6.

### Species-specific PCR assay

All the above 86 DNA samples were further subjected to PCR assay using species-specific primers for the ESAT-6 region. The details of the assay are given in Example 5. Briefly, the 86 DNA samples were amplified using oligonucleotide primer pair having SEQ ID NO: 3 and SEQ ID NO 4. The sequences of the primers are given below:
ESAT-6 F: 5' gcg gat ccc atg aca gag cag cag tgg a 3' (SEQ ID NO: 3) and
ESAT-6 R: 5' ccc aag ctt cct atg cga aca tcc cag tga cg 3' (SEQ ID NO: 4)

The DNA samples along with the primers, DNA polymerse in a suitable buffer were amplified. Out of 86 samples analyzed, (85 plus two types of growth in one patient) only 67 (77.9%) samples showed an amplification product of 320 bp which corresponds to *Mycobacterium tuberculosis* species-specific *esat-6* gene (See Table 1 for details). Figure 2 shows the amplification profile of some of the samples which were positive for the presence of *Mycobacterium species* based on genus-specific 16s rRNA PCR assay and other conventional method of diagnosis. It is clear from Fig. 2 that all the lanes did not show the presence of amplified product of size 320 bp. Lane 16 did not show any amplification and it corresponds to *M.bovis* strain This clearly indicate that this assay based on *esat* 6 gene is specific for *M.tuberculosis.* Table 2 gives the data of the species specific assay using different strains of *Mycobacterium.* It is clear from Table 2 and Fig. 2 that the PCR assay based on *esat-6* gene is specific for *M.tuberculosis.*

### Sequence analysis of 16Sr RNA genes

The patient, from whom two types of Mycobacterial colonies grew, was on anti-tubercular treatment for the past four years but without any significant clinical benefit. Both the colonies were subjected to colony characterization following the conventional methods as well as the polymerase chain reaction and 16S rRNA-sequencing. One of the two colonies did not contain the *esat-*6 gene, though genus-specific PCR was positive. The non-tubercular colony, which was ESAT-6 PCR negative, was identified as *M. smegmatis* while the other as *M. tuberculosis* based on sequence analysis of the 16S rRNA genes. The details of the sequencing of the 16S rRNA genes are given in Example 6.The sequence of the *M smegmatis 16S* rRNA gene (Accession No: AF504932) is different from that of *M*. *tuberculosis.* The *M.tuberculosis* sequence obtained by the applicant is same as that in the Gen Bank data base.

Various environmental and human opportunistic Non-tubercular *Mycobacterium* strains were identified by gene sequencing and the sequences have been submitted to GenBank with Accession no AF419854, AF504932, AF504931, AF504926, AF498754, AF498317.

On the basis of genus and species-specific primers used in this study, the Applicants found very high rate of non-tubercular Mycobacteria in HIV infected patients (33.3%, 5/15). It was closely followed by patients with lymphedenitis (29.1%, 7/24) and patients unresponsive to antitubercular treatment (20.8%, 5/24). These findings were highly significant (p <0.001) while comparing the incidence of non-tubercular Mycobacteria in all the above groups with prevalence in new cases (4.5%). All the 18 non-tubercular Mycobacteria have been identified phenotypically and genotypically. The 16S.rRNA gene was amplified from all these strains and sequenced to confirm the absence of M.tuberculosis. in the samples.

The All India Institute of Medical Sciences (AIIMS), New Delhi is a tertiary care hospital and most of the patients are already treated for tuberculosis empirically at local peripheral level. As a result less number of cases visit this hospital directly without taking prior treatment. As our figures show, more than half (52.2%) of these patients were taking treatment but without improvement. The prevalence of non-tubercular Mycobacteria in these patients was significantly (p <0.001) higher than new cases (20.8% vs. 4.5%). This finding is in concurrence with another major study published recently from south India (21). In this study, the prevalence of MDR in newly diagnosed cases of pulmonary tuberculosis was 2.5% white in previously treated cases it was 81.2% of the *M. tuberculosis* isolates (21). Our study also indicates that 79.2% cases were MDR-*M. tuberculosis* while the rest 20.8% cases did not respond because the infection was caused by non-tubercular Mycobacteria.

As expected, in the present study, the prevalence of non-tubercular Mycobacteria was very high in AIDS associated pulmonary tuberculosis. In another study (5) carried out from south India on HIV-TB co-infection, recently showed that in AIDS associated tuberculosis the primary unresponsiveness to anti-tubercular treatment was as high as 33.9%. We also found that our 33.3% patients had non-tubercular Mycobacteria, most of them identified to be *Mycobacterium avium-intracellulare* using 16s rRNA sequencing See Example (6). Since the non-tubercular Mycobacteria are not susceptible to conventional anti-tubercular treatment, it is always desirable to identify these isolates up to the species level, particularly from drug resistant cases. Though, the earlier study from south India (5) did not specify the causative species from unresponsive AIDS patients, our findings are another milestone in this direction, clearly emphasizing the need for speciation of the causative species of Mycobacteria. The present study countermands the general belief that all non-responding cases in India are due to MDR-TB. Our data indicates that more than one quarter of these patients who do not respond to primary ATT are infected with non-tubercular Mycobacteria.

*M. tuberculosis* has been reported as a leading cause of lymphadenitis in developing world and the USA (26, 33). The data from India is scarce and only a few case reports are available and the speciation is based only on phenotypic features which have several limitations. The high rate (29.2%) of non-tubercular Mycobacteria causing lymphadenitis in the present study could, therefore, be explained on the basis of highly sensitive and *M. tuberculosis* specific molecular method used here. Also most of the non-tubercular Mycobacterium species were identified as *Mycobacterium avium* complex based on 16s rRNA sequencing as given in Example 6. *Mycobacterium* strains were identified by gene sequencing of the 16S rRNA region and the sequences have been submitted to GenBank with Accession no AF419854, AF504932, AF504931, AF504926, AF498754, AF498317

The Mycobacterial DNA detection rate in a multicentric study carried out by these claimants was found to be between 90-96% on all the sputum samples taken from confirmed cases of pulmonary tuberculosis and no false positive were observed as given in Example 7.

The present invention teaches on specific method for detection of *M.tuberculosis.* This invention teaches that the ESAT-6 premiers can be used as rapid and accurate method of identifying the *Tuberculosis* from the *mycobacterium* isolates obtained either conventional or rapid culture techniques. This will obviate the need of performing several biochemical methods, which are time consuming, and often not reproducible. The table (1) clearly proves the superiority of esat-6 primers over the biochemical method of speciation where 4 isolates were incorrectly identified as *M.tuberculosis but* finally identified as non-tubercular mycobacteria. We also propose that these primers can be used to diagnose the tubercular infections, directly on the clinical samples. However, when non-tubercular mycobacterial diseases are expected in higher percentage such as in patients with HIV infection, patients not responding to standard anti-tubercular treatment and in Mycobacterial lymphadenitis, these primers should invariably be combined with genus specific primers.

The invention is further exemplified with the following examples which are provided to a person average skill in the art and these examples are not considered to limit the scope of the invention.

### EXAMPLES

### Example 1

### Clinical Samples:

Clinical samples were obtained from patients attending the out patient department (OPD), or hospitalized patients at the All India Institute of Medical Sciences (AIIMS), New Delhi, India. All the samples were processed in the Clinical Microbiology Division, Department of Laboratory Medicine. A total of 3072 patients who attended the general OPD of AIIMS, with complaints of fever, cough and expectoration were investigated to rule out tuberculosis, as a routine work-up. However, only 265 of these patients had clinico-radiological findings suggestive of tubercular etiology and were referred to specialized clinics. These referred patients fulfilled the inclusion criteria as per CDC guidelines and were included in this study. After informed consent of the patients, their routine investigations were carried out and their blood samples were also tested for human Immunodeficiency virus (HIV) infection. The clinical samples investigated for Mycobacterial isolation included sputum, lymph node aspirate or biopsy specimens and pleural fluid, according to the clinical manifestations. In case of sputum, 1-3 samples from each patient were obtained, while for other specimens sampling was done only once. However, repeat samples from the same patient were not counted as different, even if one or all the three samples from one patient were culture positive, it was counted as one only. All samples were processed on the same day of receipt. The sputum samples were decontaminated and concentrated using Petroff's method (12) within 24 hours of receipt. The decontaminated samples were inoculated on L-J culture slants. One aliquot of the pellet was stored at -20°C until further use. From the remaining pellet slide smears were prepared.

### Example 2

### Microscopy and routine culture:

The slide smears were air dried and heat fixed. One set of smears was stained with auramine-O (A-O) fluorochrome staining and examined under epifluorescence at 400X using Nikon^{®™} fluorescent microscope and the another set stained with Zeihl-Neelsen staining and examined under oil emersion (1000X). The results and quantification of acid fast bacilli (AFB) were reported as per revised WHO guidelines (33): scanty (1 or 2 AFB in 300 oil fields), 1+ (3-9 AFB in 100 fields), 2+ (1-10 AFB per oil field), or 3+ (10 or more AFB per oil field).

Culture and identifications were made according to the standard method (12, 36) by inoculating on a Lowenstein-Jensen (L-J) slant with 0.2 to 0.5 ml of the decontaminated sputum sample. Cultures were incubated at 37°C till the growth or for 8 weeks whatsoever was early. The cultured Mycobacteria were identified by staining and standard biochemical methods including Niacin, heat stable catalase, aryl sulphatase and Tween-80 hydrolysis tests.

### Example 3

### Extraction of genomic DNA:

The DNA was isolated directly from the *Mycobacterium* cultures by a procedure which the applicants have been following in the laboratory for the last several years. Briefly: 2-3 loopful (∼100mg) of Mycobacterial cultures was transferred to an eppendorf tube containing 200 µl of sterile distilled water. The suspension was incubated at 80°C in a water bath for 20 minutes. To the suspension 200 µl chloroform was added followed by vortexing. The suspension was again incubated at 65°C for 10 minutes and centrifuged at 9000 rpm for 2 minutes. The clear supernatant containing Mycobacterial DNA was taken for assay.

### Example 4

### Genus-specific PCR Assay:

Mycobacterial DNA (50ng) was amplified in a 50 µl reaction mixture for the amplification of 16S rRNA gene The reaction mixture contained, 200µM deoxynucleoside triphosphates, dNTPs (Bangalore Genei, Pvt. Ltd., Bangalore, India), 5µl of 10X buffer [100mM TAPS (pH 8.8), 15mM MgCl₂, 500mM KCl and 0.1% gelatin] and 1.5 units of *Taq* DNA polymerase. The working concentration of each primer was 0.5µM. The temperatures used were: 94°C for 5 minutes; then 25 to 35 cycles of 94°C for 1 minute, 62°C for 2 minutes and 72°C for 2 minutes. A total 40 amplification cycles were carried out followed by final extension at 72°C for 10 minutes. The PCR products were resolved on a 0.9% agarose gel after ethidium bromide staining. The amplicons of 1030 bp size indicated positive result (Figure 1). Primers for the amplification of a 1030 bp 16S rRNA gene were taken, as described elsewhere (14, 15, 28, and 33). The primers for16S rRNA was:
Primer 16S rRNA 285: 5' gag agt ttg atc ctg get cag 3' (SEQ ID NO 1) and
Primer 16S rRNA 264: 5' tgc aca aca ggc cac aag gga 3. (SEQ ID NO 2)

### Example 5

### Species- specific PCR Assay:

All the samples were also subjected to *M. tuberculosis* specific PCR, using primers designed to amplify full length *esat-*6 (Rv3875) gene. These novel primers have been designed for the first time for species-specific PCR based diagnosis of *Mycobacterium tuberculosis.* The primers also contained restriction sites for cloning in an expression vector. The primers were:
ESAT-6F: 5' gcg_gat ccc atg aca gag cag cag tgg a 3' (Bam HI site underlined) (SEQ ID NO 3) and
ESAT-6R ccc aag ctt cct atg cga aca tcc cag tga cg 3' (Hind III site underlined) (SEQ ID NO 4)

Mycobacterial DNA (50ng) was amplified in a 50 µl reaction mixture for the amplification of the *esat-*6 gene The reaction mixture contained, 200µM deoxynucleoside triphosphates, dNTPs (Bangalore Genei, Pvt. Ltd., Bangalore, India), 5µl of 10X buffer [100mM TAPS (pH 8.8), 15mM MgCl₂, 500mM KCl and 0.1% gelatin] and 1.5 units of *Taq* DNA polymerase. The working concentration of each primer was 0.5µM. The temperature cycles used were: 30 cycles of 1 min at 94°C, 1 min at 65°C, and 1 min at 72°C; followed by final extension at 72°C for 10 min The PCR products were resolved on a 0.9% agarose gel after ethidium bromide staining. The products were electrophoreses in 1.5% agarose gels, to resolve the PCR product of 320bp (Figure 2) Further the PCR amplified fragment obtained using primers having SEQ ID NO 3 and SEQ ID NO.4 from genomic DNA of a local clinical isolate of *M.tuberculosis* was cloned in a plasmid vector and sequenced. The nucleotide sequence obtained was identical to that published by Cole *et al* (4). The sequence has been deposited with the GenBank with accession no. AF420491.

### Example 6: Sequencing of 16S r RRNA genes of Mycobacteria species

The PCR fragment obtained using primers (SEQ.ID.NO.1 AND 2) from genomic DNA of a local clinical isolate of *M. tuberculosis* was cloned into an intermediate vector pGEM-T easy and its nucleotide sequence ascertained. Several of the clinical samples which showed positive amplification after the species-specific amplification were analyzed using the sequencing of the 16S rRNA genes to confirm the presence of *Mycobacteria tuberculosis.* The clinical samples which did not show amplification using primers based on *esat-6* gene were analysed using the 16S rRNA gene sequences. It was clear that the samples which did not show amplification with *esat-6* gene were not Mycobacteria *tuberculosis.* Most of them were identified to be *Mycobacterium avium-intracellulare* based on 16S. rRNA sequences. The various Non-tubercular Mycobacteria were identified by gene sequencing and the sequences have been submitted to GenBank with Accession No.AF419854, AF504932, AF504931, AF504926, AF498754, and AF498317. Since the Non-tubercular mycobacteria are not susceptible to conventional anti-tubercular treatment, it is always desirable to identify these isolates upto species level, particularly from drug resistant cases.

### Example 7: Detection of Mycobacterium tuberculosis from confirmed clinical samples

From the clinical study undertaken, 50 clinical samples were used for detection of Mycobacterium. All the above 50 sputum samples taken were from confirmed cases of pulmonary tuberculosis. The sputum samples were subjected to two PCR amplifications as given in Example 4 and 5 The PCR amplified Products were separated on agarose gel as given in example 4 and 5..The amplified products were detected by staining with ethidium bromide. Most of the 50 samples showed amplification of a DNA product of 320bp in length. The data from the clinical samples is shown in Table 3

### Example 8: Detection of different species of Mycobacteria

The PCR assay was also conducted on different species of *Mycobacteria.* The different Mycobacterial strains used in this study are shown in Table 2. DNA samples from the different strains were isolated from cultures as given in Example 3. The various DNA samples were subjected to both genus-specific and species-specific PCR assay. The genus - specific assay was carried out using 16S rRNA primers having SEQ ID NO: 1 and 2 as given in Example 4. The species-specific assay was carried out using species-specific primers having SEQ ID NO: 3 and SEQ ID NO: 4 as given in Example 5. The PCR amplified products were separated on agarose gels and analyzed. The data from these experiments is shown in Table 2.

Table 2 clearly shows that the *esat-6* species-specific primers were highly specific for *M. tuberculosis.* Even though primers specific for *Mycobacterium tuberculosis* complex (which includes *M. bovis)* have been reported, ours is the first invention which can differentiate *Mycobacterium tuberculosis from M .bovis.* It is important to differentiate *Mycobacterium tuberculosis from M.bovis* as *M.bovis* can be prevalent due to post BCG vaccination infection. In India, BCG (Bacillus of Calmette and Guerin) vaccine is administered in early childhood to all children. The vaccine contains mutated BCG strain of *M. bovis.* So far there are no rapid and direct molecular methods of making exclusive diagnosis of *M. tuberculosis infection* minus *M. bovis.* Moreover, the limitation of antibody detection assays using ESAT-6 antigen is enormous in India as stated earlier.. The *Mycobacterium bovis* is also an important cause of tuberculosis in animals (cattle) of India and traditionally the Indian farmers who consume raw cow milk will have antibodies against these bacteria. Therefore the antibody detection methods will give false positive report of tuberculosis in otherwise healthy individuals and many patients may undergo unnecessary anti-tuberculosis treatment while not treating the actual disease. Therefore, our invention will revolutionize the *M. tuberculosis* species-specific diagnosis by using multiplex PCR (using 16S rRNA and *esat-6* primers) directly on the clinical samples or using single *esat-6* primers on the culture isolates. 1.

**Table 1. Detection rate of Mycobacterial etiology in 4 patient groups using various conventional and molecular methods**

| Patent Culture | Biochemical | | | PCR identification | | | |
|---|---|---|---|---|---|---|---|
| Group | isolates | identification | | 16S rRNA | | *Esat-*6 | |
| | | M.tb | NTM | + | - | + | - |
| AIDS | 15 | 10 (66.6%) | 5(33.3%) | 15 | 0 | 8 | 7 |
| Lymphadenitis | 24 | 19 (79.2%) | 5 (20.8%) | 24 | 0 | 17 | 7 |
| MDR-TB | 24+1* | 19 (76.0%) | 6 (24.0%) | 25 | 0 | 19 | 6 |
| Rx Naïve | 22 | 21 (95.5%) | 1(4.5%) | 22 | 0 | 21 | 1 |
| Total | 85+1 * | 69 (81.2%) | 16 (18.8%) | 85 | 0 | 65 | 20 |

| | | | | | | | |
|---|---|---|---|---|---|---|---|
| *from one patient, two types of isolates were obtained. One was identified as *M*. *tuberculosis* and the other as *M. smegmatis.* | | | | | | | |

**Table 2**

| Mycobacterial species | 16S rRNA PCR result | 6 PCR result |
|---|---|---|
| | | |
| *M. tuberculosis* | + | + |
| *M. bovis (BCG)* | + | - |
| *M. avium* | + | - |
| *M. smegmatis* | + | - |
| *M. duvalli* | + | - |
| *M. fortuitum* | + | - |
| *M. celatum* | + | - |
| *M. austroafricanum* | + | - |
| *M. flavescence* | + | - |

**Table 3: The relative sensitivity of various diagnostic methods**

| N=50 | Smear | Culture | 16srRNA | ESAT-6 |
|---|---|---|---|---|
| Positive | 18 | 31 | 48 | 45/48 |
| Negative | 32 | 19 | 10 | 3/48* |

| | | | | |
|---|---|---|---|---|
| *None of the 16SrRNA PCR negative sample was found ESAT-6 PCR positive | | | | |

### References

1. Boddinghaus, B., T. Rogall, T. Flohr, H. Blocker, and E.C. Bottger. 1990. Detection and identification of Mycobacteria by amplification of rRNA. J. Clin. Microbiol. 28: 1751-1759.
2. Bottger, E.C., A. Teske, P. Kirschner, S. Boost, H.R.Chang, V.Beer, and B. Hirschel. 1992. Disseminated Mycobacterium genavense infection in-patients with AIDS. Lancet. 340:76-80.
3. Cardoso, F.L., P.R. Antas, A.S. Milagres, A. Geluk, K.L. Franken, E.B. Oliveira, H.C. Teixeira, S.A. Nogueira, E.N. Sarno, P. Klatser, T.H. Ottenhoff, E.P. Sampaio. 2002. T-cell responses to the Mycobacterium tuberculosis-specific antigen ESAT-6 in Brazilian tuberculosis patients. Infect. Immun. 70(12):6707-6714.
4. Cole ST, Brosch R, Parkhill J, et al. 1998.Deciphering the biology of Mycobacterium tuberculosis from the complete genome sequence. Nature. 393(6685):537-544.
5. Deivanayagam, C.N., S. Rajasekaran, R. Venkatesan, A. Mahilmaran, P.R. Ahmed, S. Annadurai, S. Kumar, C. Chandrasekar, N. Ravichandran, and R. Pencillaiah. 2002. Prevalence of acquired MDR-TB and HIV co-infection. Indian J. Chest Dis. Allied Sc. 44: 237-242.
6. Devallois, A., M. Picardeau, K.S. Goh, C. Sola, V. Vincent, and N. Rastogi. 1996. Comparative Evaluation of PCR and commercial DNA probes for detection and identification to species level of Mycobacterium avium and Mycobacterium intracellulare. J. Clin. Microbiol. 34:2756-22759.
7. Djuretic, T., J. Herbert, F. Drobniewski, et al. 2002. Antibiotic resistant tuberculosis in the United Kingdom: 1993-1999. Thorax. 57(6):477-482.
8. Doherty TM, Demissie A, Olobo J, Wolday D, Britton S, Eguale T, Ravn P, and Andersen P. 2002. Immune responses to the Mycobacterium tuberculosis-specific antigen ESAT-6 signal sub clinical infection among contacts of tuberculosis patients. J. Clin. Microbiol. 40(2):704-706
9. Fiss, E., F.Chehab and G.Brooks, 1992. DNA amplification and reverse dot blot hybridization for detection and identification of Mycobacteria to the species level in the clinical laboratory. J. Clin. Microbiol. 30:2969-2973.
10. Horsburg, D.R. 1991. Mycobacterium avium complex infection in the acquired immunodeficiency syndrome. N.Engl. J. Med. 324:1332-1338.
11. Kapur, V., L.L. Li, M.R. Hamrick, B.B. Pilkayatis, T.M.Shinnick, A. Telenti, W.R.Jacobs, A.Banarjee, S. Cole, K.Y. Yuen, J.E. Claridge, B.N. Kreiswirth, and J.M.Musser. 1995. Rapid Mycobacterium species assignment and unambiguous identification of mutations associated with antimicrobial resistance in Mycobacterium tuberculosis by automated DNA sequencing. Arch. Pathol. Lab. Med. 119:131-138.
12. Kent, P.T., and G.P. Kubica. 1985. Public Health Mycobacteriology. A guide for level III laboratory. Center for Disease Control, Atlanta, Ga.
13. Khatri, G.R,, and T.R. Frieden. 2000. The status and prosepects of tuberculosis control in India. Int. J. Tuberc. Lung Dis. 4: 193-200.
14. Kim, B.-J., S.-H.Lee, M.A-A. Lyu, S.-J. Kim, G.-H.Bai, S.-J.Kim, G.-T.Chao. E.C. Kim, C.-Y. Cha and Y.-H. KooK.1999. Identification of Mycobacteria by comparative sequence analysis of the RNA polymerase gene (rpo B). J. Clin. Microbiol, 37: 1714-1720.
15. Kirschner, P., B. Springer, Uvogel, A Meier, A Wrede, M , Liekenbeck, F.-C. Bange, and E.C. Bottger, 1993. Genotypic identification of Mycobacteria by nucleic acid sequence determination report of a 2 year experience in a clinical laboratory. J.Clin. Microbiol. 29:1596-1603
16. Kirschner, P., J. Rosenau, B. Springer, K. Teschner, K. Feldmann and E.C. Bottger. 1996. Diagnosis of Mycobacterial Infections by Nucleic acid amplification: 18-month prospective study. J.Clin Microbiol. 34:304-312.
17. Lee, H., H. J. Park., S. N. Cho., G. H. Bai and A.J. Kim. 2000. Species identification of Mycobacteria by PCR- Restriction Fragment length Polymorphism of the rpoB gene. J. Clin. Microbiol. 38:2966-2971.
18. Levy-Frebault V., B. Pangon, A. Bure , C. Katlama, C. Marche, and H.L. David. 1987. Mycobacterium simiae and Mycobacterium avium-M. intracellulare mixed infection in acquired immune deficiency syndrome. J. Clin. Microbiol. 25:154-7
19. Moro, M.L., A. Gori, I.Errante, A. Infuso, et al. 1998. An outbreak of multi drug resistant tuberculosis involving HIV infected patients of two hospitals in Milan, Italy. AIDS. 12: 1095-1102.
20. Murcia-Aranguren, M.I., J.E. Gomez-Marin, F.S. Alvarado, J.G. Bustillo, de E. Mendivelson, B. Gomez, C.I. Leon, W.A. Triana, E.A.Vargas, E. Rodriguez. 2001. Frequency of tuberculous and non-tuberculous Mycobacteria in HIV infected patients from Bogota, Colombia. BMC Infectious Diseases. 1:21
21. Paramasivan, C.N., P. Venkataraman, V. Chandrasekaran, S. Bhat S, and P.R.Narayanan PR. 2002. Surveillance of drug resistance in tuberculosis in two districts of South India. Int. J. Tuberc. Lung Dis. 6(6):4794-4784
22. Patel, J.B., D.G.B. Leonard, X. Pai, J. M. Musser, R.E. Berman, and I. Nachamkin. 2000. Sequence based identification of Mycobacterium species using the MicroSeq 500 16S rDNA Bacterial Identification System. J. Clin. Microbiol. 38:246-251.
23. Pollock, J.M., J. McNair, H. Bassett, J.P. Cassidy, E. Costello, H. Aggerbeck, I. Rosenkrands, P. Andersen. 2003. Specific Delayed-Type Hypersensitivity Responses to ESAT-6 Identify Tuberculosis-Infected Cattle. J. Clin. Microbiol. 41(5):1856-1860
24. Progress toward Tuberculosis Control-India 2001- 2002. MMWR. 51 (11): 229-232.
25. Pym AS, P. Brodin, L. Majlessi, R. Brosch, C. Demangel, A. Williams, K.E. Griffiths, G. Marchal, C. Leclerc, S.T.Cole. 2003. Recombinant BCG exporting ESAT-6 confers enhanced protection against tuberculosis. Nat. Med. 9(5):533-539
26. Ratanasuwan, W., W. Techasathit, V. Chuenarom, S. Suwanagool, T. Anekthananont, J. Jearanaisilavong, A. Chaiprasert. 2002. Infection due to nontuberculous Mycobacterium other than MAC in AIDS patients at Siriraj hospital during 1998-2000: saprophyte vs pathogen. J. Med. Assoc. Thai. 85(8):886-893
27. Rogall, T, T flohr, and E. Bottger. 1990. Differentiation of Mycobacterial species by direct sequencing of amplified DNA. J. Gen. Microbiol. 136:1913-1920.
28. Roth, A., U.Reischl, A. Streubel, L.Naumann, R.M. Kroppenstedt, M.Habicht, M. Fischer and H. Mauch. 2000. Novel diagnostic Algorithm for identification of Mycobacteria using Genus-specific Amplification of the 16S-23S rRNA gene spacer and Restriction Endonucleases. J. Clin. Microbiol. 38:1094-1104.
29. Shahdad S. 2002. Sequence based species identification of Mycobacteria. Ph.D. Thesis. All India Institute of Medical Sciences, New Delhi.
30. Shinnick.T.M., and R.C.Good. 1994. Mycobacterial Taxonomy. Eur. J. Clin. Microbiol. Infect. Dis.13:884-901.
31. Singh S. 2000. Diagnosis of Human Immunodeficiency virus infection and AIDS. P 51-90. IN: Proceeding of V Round Table Conference Series. No. 6. Gupta S & Sood OP (Ed). Ranbaxy Science Foundation, New Delhi.
32. Singla, R., S. Gupta, R. Gupta, V.K. Arora. 2001. Efficacy and safety of sparfloxacin in combination with kanamycin and ethionamide in multi drug-resistant pulmonary tuberculosis patients: preliminary results. Int. J. Tuberc. Lung Dis. 5 (6): 559-563.
33. Springer. B., L.Stockman, K.Teschner, G.D. Roberts, and E.C. Bottger. 1996. Two laboratory collaborative study on identification of Mycobacteria; molecular versus phenotypic methods. J. Clin. Microbiol. 34: 296-303.
34. Wallace. R.J.1994. Recent changes in taxonomy and disease manifestations of the rapidly growing Mycobacteria. Eur. J. Clin. Microbiol. Infect. Dis.13:953-960.
35. World Health Organization. 1998. Part II. Microscopy, p.77. Laboratory services in tuberculosis control. World Health Organization, Geneva, Switzerland.s

## Claims

1. An oligonucleotide primer pair consisting of SEQ ID NO: 3 and SEQ ID NO: 4 for amplification of Early Secretory Antigenic Target *(esat)-*6-gene of *Mycobacterium* species.

2. A method for detecting *M.tuberculosis* in a sample based on the amplification of *esat-6* gene, the said method comprising the steps of :
a) isolating DNA template from the sample,
b) amplifying the DNA template by adding a reaction buffer, oligonucleotide primer pair consisting of SEQ ID NO: 3 and SEQ ID NO: 4, and heat stable DNA polymerase to obtain an amplified DNA product, and
c) subjecting the amplified DNA product of step (b) to separation, and staining to detect the presence of amplified DNA product wherein the presence of amplified DNA product is indicative of *Mycobacterium tuberculosis* in the sample.

3. A method according to claim 2, wherein the sample is either clinical sample or culture sample.

4. A method according to claim 3, wherein the clinical samples is selected from a group of sputum, bronchoalveolar, lavage fluid, pleural fluid, ascetic/peritoneal fluid, cerebrospinal fluid (CSF), pus fecal matter, urine, amniotic fluid, menstrual blood, peripheral blood or other body fluids, lymph node, pus or other aspirate, and tissue biopsies.

5. A method as claimed in 2 wherein in step (b) the amplification is by polymerase chain reaction.

6. A method as claimed in 2 wherein the amplification consists of 25-35 cycles of amplification.

7. A method according to claim 2, wherein in step (b) the heat stable DNA polymerase is *Taq polymerase.*

8. A method as claimed in 2 wherein in step (c) the separation is done preferably by gel electrophoresis.

9. A method as claimed in 2 wherein in step (c) the staining is by ethidium bromide.

10. A method as claimed in 2 wherein in step (c) the amplified DNA product is 320 base pair in length.

11. A diagnostic kit for detection of *Mycobacterium tuberculosis,* from other species of *Mycobacteria* comprising oligonucleotide primers consisting of SEQ ID NO: 3 and SEQ ID NO: 4, all four deoxyribonucleotide triphosphates (dNTPs), reaction buffer, *Taq polymerase,* DNA marker, and positive and negative control.

12. A method for detecting *M tuberculosis* based on amplification wherein, the said method comprising the steps of:
i. amplifying the 16s rRNA region from the isolated DNA template using the primer pair consisting of SEQ ID NO: 1 and SEQ ID NO: 2 to obtain first amplified product using conventional method,
ii. detecting the amplified product of step (a) wherein the presence of 1030 base pair amplified DNA product is indicative of positive sample for the presence of *Mycobacterium* species,
iii. employing the DNA from the positive samples identified from step (b) for further detection *of M. tuberculosis* based on the amplification of *esat-6* gene,
iv. amplifying the *esat-6* gene using the primer pair consisting of SEQ ID NO: 3 and SEQ ID NO: 4 to obtain second amplified product using method as claimed in claim 2, and
v. detecting the amplified product of step (d) wherein the presence of 320 base pair is indicative of *Mycobacterium tuberculosis* in the sample and absence is indicative of other *Mycobacterium* species.

13. A method according to claim 12, wherein the DNA template is obtained either from clinical sample or from culture sample.

14. A method according to claim 13, wherein the clinical sample is selected from a group of sputum, bronchoalveolar, lavage fluid, pleural fluid, ascetic/peritoneal fluid, cerebrospinal fluid (CSF), pus fecal matter, urine, amniotic fluid, menstrual blood, peripheral blood or other body fluids; lymph node, pus or other aspirate, and tissue biopsies.

15. A method as claimed in 12 wherein the amplification is by polymerase chain reaction.

16. A method according to claim 15 wherein the amplification is by heat stable DNA polymerase such as *Taq polymerase.*

17. A method as claimed in 12 wherein in step (i) the amplification consists of 30-40 cycles of amplification.

18. A method as claimed in 12 wherein in step (iii) the amplification consists of 25-35 cycles of amplification

## Patentansprüche

1. Oligonukleotid-Primerpaar bestehend aus SEQ ID NO:3 und SEQ ID NO:4 zur Amplifikation von Early Secretory Antigenic Target (*esat*)-6-Gen von *Mycobacterium-*Spezies*.*

2. Verfahren zum Nachweis von *M*. *tuberculosis* in einer Probe basierend auf der Amplifikation des *esat*-6-Gens, wobei das Verfahren die Schritte umfasst:
a) Isolieren von DNA-Templat aus der Probe,
b) Amplifizieren des DNA-Templats durch Zugabe eines Reaktionspuffers, eines Oligonukleotid-Primerpaars bestehend aus SEQ ID NO:3 und SEQ ID NO:4 und hitzestabiler DNA-Polymerase, um ein amplifiziertes DNA-Produkt zu erhalten,
c) Auftrennen des amplifizierten DNA-Produkts aus Schritt (b) und Anfärben, um die Anwesenheit von amplifiziertem DNA-Produkt nachzuweisen, wobei die Anwesenheit von amplifiziertem DNA-Produkt *Mycobacterium tuberculosis* in der Probe anzeigt.

3. Verfahren nach Anspruch 2, wobei die Probe entweder eine klinische Probe oder eine Probe aus einer Kultur ist.

4. Verfahren nach Anspruch 3, wobei die klinische Probe ausgewählt ist aus der Gruppe bestehend aus Sputum, bronchoalveolärer Spülflüssigkeit, Pleuraflüssigkeit, Aszites-/Peritonealflüssigkeit, Zerebrospinalflüssigkeit (CSF), Eiter, Fäkalmaterial, Urin, Fruchtwasser, Menstrationsblut, peripherem Blut oder anderen Körperflüssigkeiten, Lymphknoten, Eiter oder anderen Aspiraten, und Gewebebiopsien.

5. Verfahren nach Anspruch 2, wobei die Amplifikation in Schritt (b) durch Polymerasekettenreaktion erfolgt.

6. Verfahren nach Anspruch 2, wobei die Amplifikation aus 25-35 Ampifikationszyklen besteht.

7. Verfahren nach Anspruch 2, wobei die hitzestabile DNA-Polymerase in Schritt (b) *Taq-Polymerase* ist.

8. Verfahren nach Anspruch 2, wobei die Auftrennung in Schritt (c) durch Gelelektrophorese erfolgt.

9. Verfahren nach Anspruch 2, wobei das Anfärben in Schritt (c) mit Ethidiumbromid erfolgt.

10. Verfahren nach Anspruch 2, wobei das amplifizierte DNA-Produkt in Schritt (c) eine Länge von 320 Basenpaaren hat.

11. Diagnostischer Kit zur Unterscheidung von *Mycobacterium tuberculosis* von anderen *Mycobacterium-Spezies,* umfassend Oligonukleotidprimer bestehend aus SEQ ID NO:3 und SEQ ID NO:4, alle vier Desoxyribonukleotidtriphosphate (dNTPs), Reaktionspuffer, *Taq-Polymerase,* DNA-Marker und positive und negative Kontrolle.

12. Verfahren zum Nachweis von *M. tuberculosis* basierend auf Amplifikation, wobei das Verfahren die Schritte umfasst:
i). Amplifizieren der 16s-rRNA-Region des isolierten DNA-Templats unter Verwendung des Primerpaars bestehend aus SEQ ID NO:1 und SEQ ID NO:2 mit üblichen Verfahren, um ein erstes amplifiziertes Produkt zu erhalten,
ii). Nachweis des amplifizierten Produkts von Schritt (a), wobei die Anwesenheit eines amplifizierten DNA-Produkts mit 1030 Basenpaaren eine hinsichtlich *Mycobacterium*-Spezies positive Probe anzeigt,
iii). Einsetzen der DNA aus den in Schritt (b) identifizierten positiven Proben zum weiteren Nachweis von *M*. *tuberculosis* basierend auf der Amplifikation des *esat*-6-Gens,
iv). Amplifizieren des *esat*-6-Gens unter Verwendung des Primerpaars bestehend aus SEQ ID NO:3 und SEQ ID NO:4 mit dem Verfahren nach Anspruch 2, um ein zweites amplifiziertes Produkt zu erhalten, und
v). Nachweisen des amplifizierten Produkt aus Schritt (d), wobei die Anwesenheit von 320 Basenpaaren *Mycobacterium tuberculosis* in der Probe anzeigt und das Fehlen andere *Mycobacterium-Spezies* anzeigt.

13. Verfahren nach Anspruch 12, wobei das DNA-Templat entweder aus einer klinischen Probe oder einer Probe aus einer Kultur erhalten ist.

14. Verfahren nach Anspruch 13, wobei die klinische Probe ausgewählt ist aus der Gruppe bestehend aus Sputum, bronchoalveolärer Spülflüssigkeit, Pleuraflüssigkeit, Aszites-/Peritonealflüssigkeit, Zerebrospinalflüssigkeit (CSF), Eiter, Fäkalmaterial, Urin, Fruchtwasser, Menstrationsblut, peripherem Blut oder anderen Körperflüssigkeiten, Lymphknoten, Eiter oder anderen Aspiraten, und Gewebebiopsien.

15. Verfahren nach Anspruch 12, wobei die Amplifikation durch Polymerasekettenreaktion erfolgt.

16. Verfahren nach Anspruch 15, wobei die Amplifikation mittels hitzestabiler DNA-Polymerase wie *Taq-Polymerase* erfolgt.

17. Verfahren nach Anspruch 12, wobei die Amplifikation in Schritt (i) aus 30-40 Ampifikationszyklen besteht.

18. Verfahren nach Anspruch 12, wobei die Amplifikation in Schritt (iii) aus 25-35 Ampifikationszyklen besteht.

## Revendications

1. Paire d'amorces oligonucléotidiques constituée de SEQ ID NO : 3 et SEQ ID NO . 4 pour l'amplification du gène *esat-6* (early secretory antigenic target ou cible antigénique sécrétée précocement) de l'espèce *Mycobacterium.*

2. Procédé de détection de *M. tuberculosis* dans un échantillon basé sur l'amplification du gène *esat*-6, ledit procédé comprenant les étapes consistant à :
a) isoler l'ADN matrice à partir de l'échantillon,
b) amplifier l'ADN matrice en ajoutant un tampon de réaction, une paire d'amorces oligonucléotidiques constituée de SEQ ID NO : 3 et SEQ ID NO : 4, et une ADN polymérase thermostable pour obtenir un produit d'ADN amplifié, et
c) soumettre le produit d'ADN amplifié de l'étape (b) à une séparation et à une coloration pour détecter la présence du produit d'ADN amplifié où la présence du produit d'ADN amplifié est indicative de *Mycobacterium tuberculosis* dans l'échantillon.

3. Procédé selon la revendication 2, dans lequel l'échantillon est soit un échantillon clinique soit un échantillon de culture.

4. Procédé selon la revendication 3, dans lequel l'échantillon clinique est choisi dans un groupe de crachats, de liquide de lavage broncho-alvéolaire, de liquide pleural, de liquide d'ascite / péritonéal, de liquide céphalorachidien (LCR), de matières fécales purulentes, d'urine, de liquide amniotique, de sang menstruel, de sang périphérique ou d'autres liquides corporels, de ganglion lymphatique, de pus ou d'un autre aspirat, et de biopsies tissulaires.

5. Procédé selon la revendication 2, dans lequel dans l'étape (b), l'amplification est réalisée par réaction en chaîne de la polymérase.

6. Procédé selon la revendication 2, dans lequel l'amplification est constituée de 25 à 35 cycles d'amplification.

7. Procédé selon la revendication 2, dans lequel dans l'étape (b), l'ADN polymérase thermostable est la *Taq* polymérase.

8. Procédé selon la revendication 2, dans lequel dans l'étape (c), la séparation est réalisée de préférence par électrophorèse sur gel.

9. Procédé selon la revendication 2, dans lequel dans l'étape (c), la coloration est réalisée par bromure d'éthidium.

10. Procédé selon la revendication 2, dans lequel dans l'étape (c), le produit d'ADN amplifié est long de 320 paires de bases.

11. Kit de diagnostic pour la détection de *Mycobacterium tuberculosis,* à partir d'autres espèces de Mycobactéries comprenant des amorces oligonucléotidiques constituées de SEQ ID NO : 3 et SEQ ID NO : 4, les quatre désoxyribonucléotides triphosphates (dNTP), du tampon de réaction, la *Taq* polymérase, un étalon d'ADN et un contrôle positif et négatif.

12. Procédé de détection de *M. tuberculosis* basé sur l'amplification, où ledit procédé comprenant les étapes consistant à :
i. amplifier la région de l'ARNr 16S à partir de l'ADN matrice isolé en utilisant la paire d'amorces constituée de SEQ ID NO : 1 et SEQ ID NO : 2 pour obtenir un premier produit amplifié en utilisant un procédé conventionnel,
ii. détecter le produit amplifié de l'étape (a) où la présence d'un produit d'ADN amplifié de 1030 paires de bases est indicative d'un échantillon positif pour la présence de l'espèce *Mycobacterium,*
iii. employer l'ADN provenant des échantillons positifs identifiés à partir de l'étape (b) pour une autre détection de *M. tuberculosis* basée sur l'amplification du gène *esat-*6,
iv. amplifier le gène *esat*-6 en utilisant la paire d'amorces constituée de SEQ ID NO : 3 et SEQ ID NO : 4 pour obtenir un second produit amplifié en utilisant le procédé selon la revendication 2, et
v. détecter le produit amplifié de l'étape (d) où la présence de 320 paires de bases est indicative de *Mycobacterium tuberculosis* dans l'échantillon et l'absence est indicative d'une autre espèce de *Mycobacterium.*

13. Procédé selon la revendication 12, dans lequel l'ADN matrice est obtenu soit à partir d'un échantillon clinique soit à partir d'un échantillon de culture.

14. Procédé selon la revendication 13, dans lequel l'échantillon clinique est choisi dans un groupe de crachats, de liquide de lavage broncho-alvéolaire, de liquide pleural, de liquide d'ascite / péritonéal, de liquide céphalorachidien (LCR), de matières fécales purulentes, d'urine, de liquide amniotique, de sang menstruel, de sang périphérique ou d'autres liquides corporels, de ganglion lymphatique, de pus ou d'un autre aspirat, et de biopsies tissulaires.

15. Procédé selon la revendication 12, dans lequel l'amplification est réalisée par réaction en chaîne de la polymérase.

16. Procédé selon la revendication 15, dans lequel l'amplification est réalisée à l'aide d'une ADN polymérase thermostable telle que la *Taq* polymérase.

17. Procédé selon la revendication 12, dans lequel dans l'étape (i), l'amplification est constituée de 30 à 40 cycles d'amplification.

18. Procédé selon la revendication 12, dans lequel, dans l'étape (iii), l'amplification est constituée de 25 à 35 cycles d'amplification.
